# EUROPEAN PATENT SPECIFICATION

(11) **EP 2 907 500 B1**
(45) Date of publication and mention of the grant of the patent: **03.04.2019**
(21) Application number: 13844768.5
(22) Date of filing: 11.10.2013
(51) Int. Cl.: A61K 8/85, A61K 8/02, A61K 8/87, A61Q 17/04, A61K 8/73, A45D 40/30, A61Q 19/00

(54) **SUN CARE COSMETIC**
SONNENPFLEGE KOSMETIK
COSMÉTIQUE DE SOINS SOLEIL

(30) Priority: 12.10.2012 JP 2012227075
(43) Date of publication of application: 19.08.2015
(73) Proprietor: Shiseido Company, Ltd., Chuo-ku Tokyo 104-0061 (JP)
(72) Inventor: KIMURA, Tomoko, Yokohama-shi Kanagawa 224-8558 (JP); KANNO, Naomi, Yokohama-shi Kanagawa 224-8558 (JP); YAMAKI, Satoshi, Yokohama-shi Kanagawa 224-8558 (JP)
(74) Representative: Ter Meer Steinmeister & Partner
(86) International application number: PCT/JP2013/077835
(87) International publication number: WO 2014/058066

(56) References cited:
- EP-A1- 2 082 869
- EP-A1- 2 191 810
- EP-A1- 2 722 169
- WO-A1-2008/129707
- WO-A1-2009/041121
- WO-A1-2013/153678
- WO-A2-2012/087517
- JP-A- H11 228 340
- JP-A- 2013 001 661
- JP-A- 2013 071 906
- JP-A- 2013 071 907
- JP-A- 2013 184 970
- Hiromi Miyazaki et al.: "An ultrathin poly(L-lactic acid) nanosheet as a burn wound dressing for protection against bacterial infection", Wound Repair and Regeneration, vol. 20 19 June 2012 (2012-06-19), pages 573-579, XP002754871, ISSN: 1524-475X Retrieved from the Internet: URL:http://onlinelibrary.wiley.com/doi/10. 1111/j.1524-475X.2012.00811.x/pdf [retrieved on 2016-02-29]
- Toshinori Fujie, Yosuke Okamura and Shinji Takeoka: "Ubiquitous transference of a freestanding polysaccharide nanosheet with the development of a nano-adhesive plaster", Advanced Materials, vol. 19 5 November 2007 (2007-11-05), pages 3549-3553, XP002754872, ISSN: 1521-4095 Retrieved from the Internet: URL:http://onlinelibrary.wiley.com/doi/10. 1002/adma.200700661/pdf [retrieved on 2016-02-29]

## Description

### FIELD OF THE INVENTION

The present invention relates to a sun care cosmetic for use in a method of protecting the skin from UV rays .

### BACKGROUND OF THE INVENTION

In the past, many expressed the desire to correct the roughness on the skin caused by pores, wrinkles, acne scars, etc.

As the external skin preparation having such a roughness correction effect, an external skin preparation for roughness correction containing a silicone-type film forming agent has been known (Patent Literature 1). In this external skin preparation for roughness correction, an excellent roughness correction effect is realized by forming a strong cosmetic film on the skin. However, there has been a problem in that the cosmetic film remains on the skin unless a cleanser for makeup cleansing is used.

Even in common makeup cosmetics and sun care cosmetics, if no cleanser for makeup cleansing is used but water, when cosmetic material is removed, there has been a problem in that cosmetic material remains in the texture and it becomes a cause of skin dullness and skin roughness.

Furthermore, an attempt has been made to provide a moisturizing effect, whitening effect, etc. by blending various active components in skin care cosmetics.

In order to increase a percutaneous absorption effect of such active components, the inclusion of the active components in a gel-sheet for moisturization (Patent Literature 2) or a functional sheet (Patent Literature 3) has been described. These sheets are gel-like and noticeable; thus keeping it on the face for a long time and going out with it was not possible.

On the other hand, when makeup cosmetics or sun care cosmetics are applied after the application of skin care cosmetics, there has been a problem in that active components may be mixed or they may be impregnated into the applicators such as brush, puff, etc.
Patent Literature 1: Japanese unexamined patent publication No. 2010-229094
Patent Literature 2: International unexamined patent publication No. 2001-31556
Patent Literature 3: International unexamined patent publication No. 2012-148980
   EP 2 191 810 A1 is directed to a makeup-assisting patch having a layer structure comprising a base layer and a pressure sensitive adhesive layer.

### DISCLOSURE OF THE INVENTION PROBLEM TO BE SOLVED BY THE INVENTION

The present invention was made in view of the above-described conventional art. The problem to be solved is to provide a sun care cosmetic being excellent in the roughness correction effect and easily removing sun care cosmetic.

### MEANS TO SOLVE THE PROBLEM

The present inventors have diligently studied to solve the above-described problems. As a result, the present inventors have found that a sun care cosmetic for use in a method of protecting the skin from UV rays, having a step of removing a support body of a thin film, subsequently a step of pasting a base material film on the skin, and subsequently a step of applying a sun care cosmetic on the base material film, wherein the thin film consists of a base material film of the thickness of 10 to 500 nm and a support body, wherein a film of hyaluronic acid, acetylated hyaluronic acid or sodium hyaluronate is supported on the base material film according to claim 1,
is excellent in the roughness correction effect and can easily remove sun care cosmetic, thus leading to the completion of the present invention. Advantageous embodiments are defined in claims 2 to 4.

### EFFECT OF THE INVENTION

The present invention can be provided a sun care cosmetic for use in a method of protecting the skin from UV rays having an excellent roughness correction effect and easily removing sun care cosmetic.

### BRIEF DESCRIPTION OF THE DRAWINGS

Fig. 1 shows the thin film used in the sun care cosmetic of the present invention.
Fig. 2 is explanatory drawings for the sun care cosmetic of the present invention.
Fig. 3 is explanatory drawings for a reference cosmetic .
Fig. 4 is explanatory drawings for the sun care cosmetic of the present invention having a step of applying a skin care cosmetic.
Fig. 5 is explanatory drawings for a reference sun care cosmetic having a step of applying a skin care cosmetic.
Fig. 6 (A) is a picture of the bare skin. Fig. 6 (B) is a picture wherein a base material film is pasted on the skin of Fig. 6 (A). Fig. 6 (C) is a picture wherein a makeup cosmetic is applied on the base material film of Fig. 6 (B).
Fig. 7 (A) is a picture of the bare skin. Fig. 7 (B) is a picture wherein a powder foundation is applied on the base material film of Fig. 7 (A).
Fig. 8 (A) is a picture of the bare skin. Fig. 8 (B) is a picture wherein a base material film is pasted on the skin of Fig. 8 (A). Fig. 8 (C) is a picture wherein a makeup cosmetic is applied on the base material film of Fig. 8 (B). Fig. 8 (D) is a picture wherein the base material film of Fig. 8 (C) is removed with cellophane tape.
Fig. 9 (A) is a picture wherein a skin care cosmetic and a makeup cosmetic are applied on the bare skin. Fig. 9 (B) is a picture wherein a base material film is pasted and a makeup cosmetic is applied after the application of a skin care cosmetic on the bare skin.
Fig. 10 (A-1) is a picture wherein a makeup cosmetic is applied on the skin. Fig. 10 (A-2) is a picture wherein the cosmetic material-covered section of Fig. 10 (A-1) is peeled off with cellophane tape (Fig. 10 (A-2') is its enlarged picture). Fig. 10 (B-1) is a picture wherein a base material film is pasted on the skin and a makeup cosmetic is applied. Fig. 10(B-2) is a picture wherein the cosmetic material-covered section of Fig. 10 (B-1) is peeled off with cellophane tape (Fig. 10 (B-2') is its enlarged picture).
Fig. 11 shows the amount of tranexamic acid in the stratum corneum collected from the skin on which only tranexamic acid solution was applied (tranexamic acid solution only) and the amount of tranexamic acid in the stratum corneum collected from the skin on which the base material film was pasted after the application of tranexamic acid solution (tranexamic acid solution + base material film) (the measurement results of three panelists).
Fig. 12 shows the amount of tranexamic acid in the stratum corneum collected from the skin on which only tranexamic acid solution was applied (tranexamic acid solution only) and the amount of tranexamic acid in the stratum corneum collected from the skin on which the base material film was pasted after the application of tranexamic acid solution (tranexamic acid solution + base material film) (average values of the measurement results).
Fig. 13 (A) is a picture of the bare skin. Fig. 13 (B) is a picture, after a lapse of 8 hours, of the skin after the application of a moisturizing lotion on the skin of Fig. 13 (A).
Fig. 14 (A) is a picture of the bare skin. Fig. 14 (B) is a picture, after a lapse of 8 hours, of the skin on which the base material film was pasted after the application of a moisturizing lotion on the skin of Fig. 14 (A).

### BEST MODE FOR CARRYING OUT THE INVENTION

At first, a thin film used in the sun care cosmetic of the present invention will be explained.

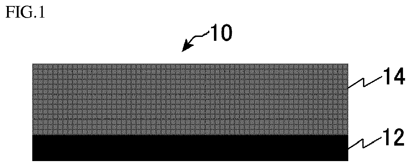

The thin film used in the present invention is shown in Fig. 1.

The thin film 10 consists of a base material film 12 with the film thickness of 10 to 500 nm and a support body 14.

It is necessary that the thickness of base material film is 10 to 500 nm. The thickness of base material film is preferably 50 to 350 nm and especially preferably 100 to 250 nm.

If the thickness of base material film is too thick, an uncomfortable feeling is caused at an application location and the roughness correction effect tends to be poor.

If the thickness of base material film is too thin, the manageability tends to be poor.

The base material film material is not limited in particular; however, it is preferable to use one or more kinds of materials selected from the group consisting of polylactic acid, polyglycolic acid, polycaprolactone, copolymers thereof, and acrylic urethane copolymer.

The preparation method of base material film is not limited in particular so far as the thickness of the present invention can be achieved. However, it is preferable to prepare by a spin-coating method, a (micro) gravure method, or a spray-coating method. In the case of spin coating method, any spin coater can be used; however, it is preferable to carry out preparation at the rotation speed of 2000 to 5000 rpm.

Moreover, a film of hyaluronic acid, sodium hyaluronate or acetylated hyaluronic acid is supported on the base material.

The thickness of the support layer (hyaluronic acid or its derivative) is not limited in particular; however, it is preferably 50 to 400 nm to produce a uniform product.

The supporting of hyaluronic acid or its derivative can be carried out by any method. The preparation of base material film can be carried out by any optional method. For example, the method wherein a solution containing hyaluronic acid or a derivative thereof is dropwise added and dried after the preparation of the base material film by a spin-coating method, a (micro)gravure method, a spray-coating method, etc.; the method wherein a dispersion liquid is dropwise added on the substrate that is used in a spin-coating method, a (micro)gravure method, a spray-coating method, etc. after obtaining the dispersion liquid in which hyaluronic acid or a derivative thereof is dispersed in the base material film material; etc. can be listed, but is not limited in particular.

As a support body, a water-soluble polymer film or a cloth is preferably used. If only the base material film is used, it is too thin, the removal after preparation is difficult, and the manageability is poor. However, a thin film excellent in manageability can be obtained by laminating the support body.

The thickness of the support body is not limited in particular, but it is preferable to be 1 to 500 µm.

The water-soluble polymer film is not limited in particular, but it is preferable to comprise one or more polymer(s) selected from the group consisting of polyvinyl alcohol or a derivative thereof, a polyether or a derivative thereof, polysaccharides, a polymer electrolyte or a salt thereof, and hyaluronic acid or a derivative thereof.

In order to make the handling easy, resin such as PET (polyethylene terephthalate) may be laminated as the support body in addition to the water-soluble polymer film when sodium hyaluronate is supported.

The cloth is not limited in particular, but a mesh or a non-woven fabric is preferable.

The mesh means a sheet of network resin, and the examples of materials include PET resin, polyester resin, nylon, and etc. From the standpoint of removability, it is preferable to use PET resin; however, either can be effectively used.

Moreover, examples of non-woven fabrics include nylon, cellulose, polyester fiber, and etc.

In the present invention, the examples of kinds of support bodies and the usage are listed to lead to the state that the base material film is uniformly pasted on the skin; however, the effect of the present patent application can be achieved so far as it can be uniformly pasted on the skin.

The sun care cosmetic for use in the method of the present invention is excellent in the roughness correction effect and is practiced to easily remove sun care cosmetics.

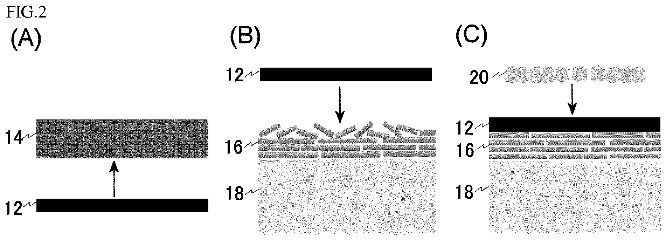

The sun care cosmetic for use in the method of the present invention has a step of removing the support body of the thin film (Fig. 2 (A)), a step of pasting the base material film on the skin (Fig. 2 (B)), and a step of applying a makeup cosmetic and/or a sun care cosmetic on the skin (Fig. 2 (C)).

At first, as shown in Fig. 2 (A), it is necessary to remove the support body of the thin film and obtain the base material film.

When a water-soluble polymer film is used as the support body, the water-soluble polymer film can be easily removed, for example, by immersing in water.

When cloth is used as the support body, the base material film can pasted on the skin and only the support body can be peeled off by applying water, lotion, etc. on the skin and pasting the base material film side of the thin film on the skin.

Subsequently, it is necessary to paste the base material film on the skin as shown in Fig. 2 (B).

When hyaluronic acid or its derivative is supported on the base material film, it is preferable that the side supporting hyaluronic acid or its derivative is pasted on the skin.

As shown in Fig. 2 (C), it is necessary to apply a sun care cosmetic 20 on the skin to which the base material film is pasted.

Sun care cosmetics are cosmetic materials used to protect the skin from UV rays, and a large amount of UV absorber is usually blended. In the present invention, sun care cosmetics are not necessarily for the body only, but the cosmetics used for the face are also included. The examples of sun care cosmetics include sunscreen, beauty essence for daytime use, milky lotion for daytime use, BB cream.

BB cream is an abbreviation of Blemish Balm cream, and it is a multifunctional cosmetic having the functions of sunscreen and beauty essence as well as the functions of makeup cosmetics such as pre-makeup, foundation, and concealer; therefore, BB cream could be classified to both makeup cosmetics and sun care cosmetics.

It is preferable that a sun care cosmetic is applied without rubbing strongly.

Furthermore, as the sun care cosmetic, it is preferable to use a cosmetic material, such as powder foundation, with a small blending quantity of liquid component. It is more preferable to use a cosmetic material with a blending quantity of liquid component of 30 mass% or less. If a cosmetic material, such as liquid foundation, with a large blending quantity of liquid component is used, the base material film may easily peel off.

When a base material film containing polylactic acid is used as the base material film, it is preferable that a large amount of a high-polarity component is not blended into a makeup cosmetic and/or a sun care cosmetic. If a large amount of a high-polarity component is blended, the film forming capability may be lowered.

The examples of high-polarity component include polyoxyethylene/polyoxypropylene dimethyl ether, phenoxyethanol, and etc.

Furthermore, it is preferable that a large amount ethanol, decamethylcyclopentasiloxane, or ethylhexyl methoxycinnamate is not blended in the makeup cosmetic and/or a sun care cosmetic. If a large amount of such a component is blended, the film forming capability may be lowered.

The sum of the above components is preferably 30 mass% or less and more preferably 10 mass% or less, and it is especially preferable that the components are not blended.

It is preferable to carry out the step of applying a sun care cosmetic on the skin after the base material film on the skin becomes dry after the step of pasting the base material film on the skin.

The standing time necessary for the base material film to become dry is not limited in particular; however, it is preferably 30 seconds or longer and more preferably 1 minute or longer.

As a reference method, the order may be reversed for the step of removing the support body of the thin film and the step of pasting the base material film on the skin.

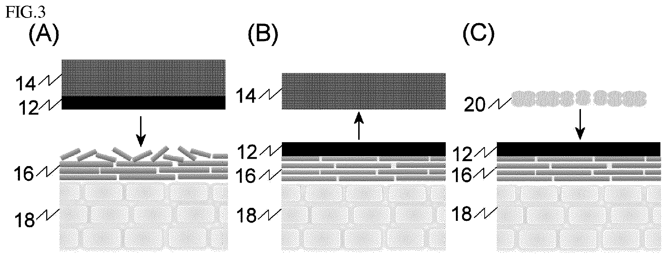

That is, the cosmetic method has a step of pasting the base material film surface of a thin film on the skin (Fig. 3 (A)), a step of removing the support body of the pasted thin film (Fig. 3 (B)), and a step of applying a makeup cosmetic and/or a sun care cosmetic on the skin (Fig. 3 (C)).

These steps can be carried out in the same way as the above embodiment.

In the step shown in Fig. 3 (A), the thin film is pasted on the moderately wetted skin. Here, the skin can be wetted with any liquid such as water or a cosmetic material. When cloth is used as the support body, the thin film can be easily pasted on such wetted skin. Thus, in this embodiment, cloth is preferably used as support body.

The base material film pasted by the cosmetic method of the present invention can be peeled off, after use, with water and other cleansers (for example, makeup remover, facial cleanser, etc.). Because the sun care cosmetics are applied on the base material film, they can be easily removed by peeling off the base material film.

Thus, the base material film, and sun care cosmetics can be removed by wetting the pasted section with even simple water. However, the base material film of the present invention is not removed by normal perspiration only.

It is preferable that the cosmetic method of the present invention is applied to the skin having roughness. By carrying out the cosmetic method of the present invention, as shown in Fig. 2 (B), an excellent roughness correction effect can be realized even for the skin having roughness in the stratum corneum 16.

It is preferable that the sun care cosmetic for use in the method of the present invention has a step of applying a skin care cosmetic prior to the step of pasting the base material film on the skin.

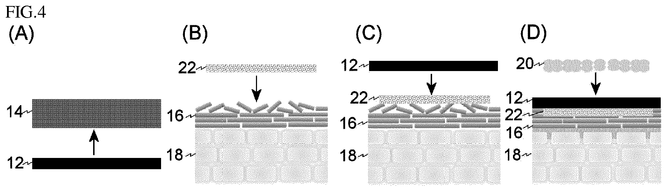

That is, it is preferable to have a step of removing the support body of the thin film (Fig. 4 (A)), a step of applying a skin care cosmetic (Fig. 4 (B)), a step of pasting the base material film on the skin (Fig. 4 (C)), and a step of applying a makeup cosmetic and/or a sun care cosmetic on the skin (Fig. 4 (D)).

As a reference method, the order for the step of removing the support body of the thin film and the step of applying the skin care cosmetic may be reversed.

That is, the order for the step of removing the support body of the thin film and the step of pasting the base material film on the skin may be reversed.

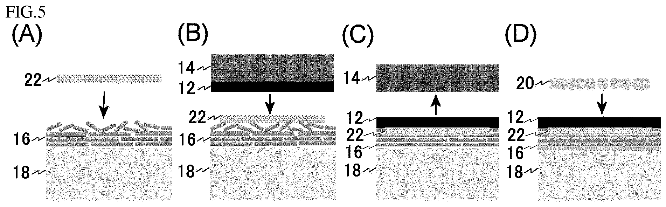

That is, the cosmetic method has a step of applying the skin care cosmetic (Fig. 5 (A)), a step of pasting the base material film surface of a thin film on the skin (Fig. 5 (B)), a step of removing the support body of the pasted thin film (Fig. 5 (C)), and a step of applying a makeup cosmetic and/or a sun care cosmetic on the skin (Fig. 5 (D)).

In the sun care cosmetic for use in the method having the step of applying a skin care cosmetic, the step of removing the support body of the thin film, the step of pasting the base material film on the skin, and the step of applying a sun care cosmetic on the skin are also carried out as explained above.

The step of applying the skin care cosmetic is shown in Fig. 4 (B) and Fig. 5 (A).

Skin care cosmetic is not limited in particular, but the examples include lotion, milky lotion, beauty essence, cream.

When cloth is used as the support body, it is preferable that the skin is wetted with the skin care cosmetic, the base material film surface of the thin film is pasted on the skin, only the cloth is peeled off, and the obtained base material film is pasted on the skin. Thus, in the embodiment shown in Fig. 5, cloth is preferably used as support body.

The skin care cosmetic present between the skin and the base material film is excellent in the percutaneous absorption effect of active components. That is, by carrying out the cosmetic method of the present invention, the amount of percutaneously-absorbed active components becomes large at the corneocytes present in the stratum corneum 16 and the epidermis 18 as shown in Fig. 4 (D). Thus, active components such as moisturizing agent, whitening agent, blood circulation promoter, plant extracts, and etc. are preferably blended in skincare cosmetic.

### EXAMPLES

Initially, the preparation method of the thin film used in the sun care cosmetic for use in the method of the present invention will be explained.

### Preparation method of thin film

All operations were carried out by setting up a spin coater (Opticoat MS-A 150, manufactured by MIKASA Co., Ltd.) inside a clean room (class: 10,000).

Silicon substrate (manufactured by KST World Corp.) was cut into 4 cm × 4 cm, immersed in SPM (H₂SO₄/H₂O₂ = 2.3:1 (v/v)) at 120°C for 10 minutes, and then washed with ion-exchanged water (specific resistance: 18 MΩcm). This substrate was placed on a spin coater, and the spin coating (4000 rpm, 20 seconds) was carried out by adding dropwise 500 µL of dichloromethane solution of poly-L-lactic acid (hereinafter referred to as PLA) (Mw: 100,000, manufactured by Polysciences Inc., 10 mg/mL) to obtain a poly-L-lactic acid film. The thickness of the obtained poly-L-lactic acid film was measured with an atomic force microscope (manufactured by Keyence Corporation); it was 120 nm.

Subsequently, 10 mg/mL sodium hyaluronate (Biohyalo 12 (manufactured by Shiseido Co., Ltd.), hereinafter referred to as HA) was prepared with 50% and 70% ethanol aqueous solutions. About 1.5 mL of this was dropwise added on the poly-L-lactic acid film and dried (80 °C, 30 minutes), the substrate surface was washed with ion-exchanged water (room temperature, 1 minute), and the surface was dried with nitrogen gas; thus a base material film (a film wherein sodium hyaluronate is supported on the poly-L-lactic acid film; the thickness: 130 nm) was obtained. The base material film is a film wherein sodium hyaluronate is supported on the poly-L-lactic acid film.

In addition, 0.5 mL of polyvinyl alcohol aqueous solution (hereinafter PVA, Mw: 22,000, manufactured by Kanto Chemical Co., Inc., 100 mg/mL) was dropwise added on the surface of the prepared base material film and dried; thus a water-soluble polymer film (PVA film) was formed on the base material film (80°C, 30 minutes). A thin film was obtained by peeling off, from the silicon substrate, the base material film together with the water-soluble polymer film.

The below-described effects were evaluated by pasting the side of supporting sodium hyaluronate on the skin with the base material film which was obtained by dissolving the water-soluble polymer film by immersing the thin film in water.

The present inventors observed the skin to which the base material film of the thin film, which is obtained by the above-described preparation method, is pasted.

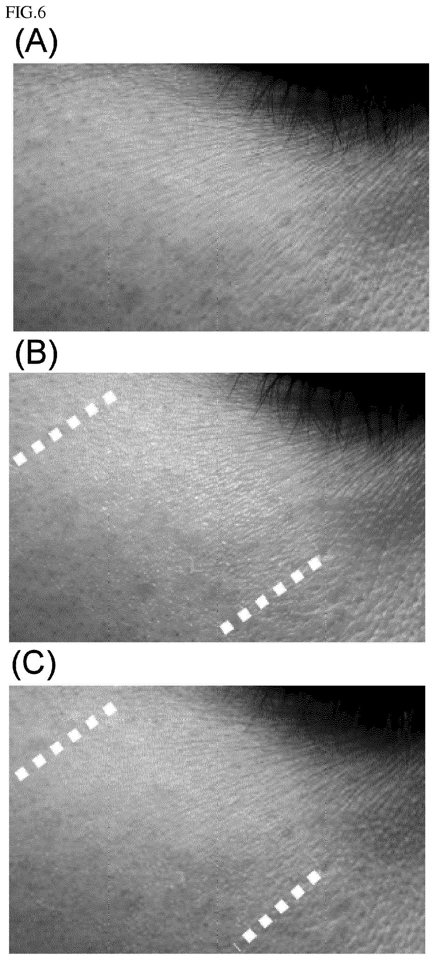

At first, a picture of the non-coated cheek of the subject was taken. The picture is shown in Fig. 6 (A).

Subsequently, a little water was put on the skin at the same location, a base material film was pasted, and a picture (section surrounded by dotted lines) was taken after waiting, until the base material film becomes dry, for about 1 minute. The picture is shown in Fig. 6 (B).

Subsequently, the powder foundation of the below-described Reference Example 1 was applied, on the skin at the same location, with a sponge in dabbing motion, and a picture was taken. The picture is shown in Fig. 6 (C).

### Reference Example 1: Powder foundation

| | |
|---|---|
| Talc | 20 mass% |
| Mica | 35 |
| Kaolin | 5 |
| Titanuim dioxide | 10 |
| Sericite | 5 |
| Coloring pigment | 5 |
| Pearlescent pigment | 10 |
| Preservative | proper quantity |
| Liquid paraffin | 8 |
| Antioxidant | 2 |

According to Fig. 6, an excellent roughness correction effect could be realized by applying the powder foundation on the skin to which the base material film was pasted.

Subsequently, the further investigation was carried out for the skin on which the base material film of the thin film, obtained by the above-described preparation method, was pasted.

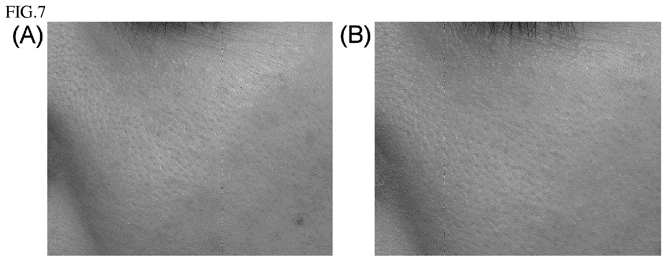

At first, a picture of the non-coated cheek of the subject is shown in Fig. 7 (A). The picture wherein the powder foundation of the above-described Reference Example 1 was applied on the skin at the same location is shown in Fig. 7 (B).

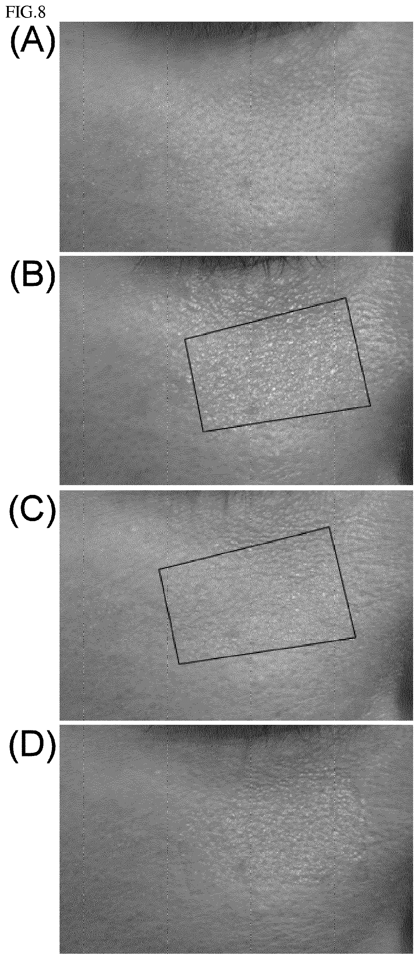

Next, a picture of the non-coated cheek of the same subject is shown in Fig. 8 (A). A little water was put on the skin at the same location, a base material film was pasted (section surrounded by lines), and a picture was taken after waiting, until the base material film becomes dry, for about 1 minute. The picture is shown in Fig. 8 (B).

Subsequently, the powder foundation of the below-described Reference Example 1 was applied, on the skin at the same location, with a sponge in dabbing motion. The picture is shown in Fig. 8 (C).

Subsequently, the pasted base material film was removed with cellophane tape. The picture is shown in Fig. 8 (D).

According to Fig. 7 and Fig. 8, it was reconfirmed that an excellent roughness correction effect could be realized by applying powder foundation on the skin to which a base material film had been pasted. Furthermore, the cosmetic material was not packed into the pores after the removal of the base material film; thus the cosmetic material could easily be removed.

Subsequently, the skin to which the base material film of different thicknesses is pasted was investigated.

At first, the present inventors prepared, by the (micro) gravure method, a thin film containing the base material film of the thickness of 330 nm (250 nm of poly-L-lactic acid film + 80 nm of acetylated hyaluronic acid). As a support body, cloth (PET mesh) was used.

After the subjects washed their faces, they were asked to apply a skin care cosmetic and a makeup cosmetic, and pictures were taken after a lapse of 5 hours. Immediately before the application of the makeup cosmetic, the base material film was pasted on only one cheek, and the support body was peeled off.

Here, as the skin care cosmetic (lotion and milky lotion) and the makeup cosmetic (pre-makeup and powder foundation), ELIXIR SUPERIEUR (both II, manufactured by Shiseido Co., LTD.) and MAQuillAGE (Perfect Multi Base BB and True Powdery, manufactured by Shiseido Co., LTD.) were used respectively.

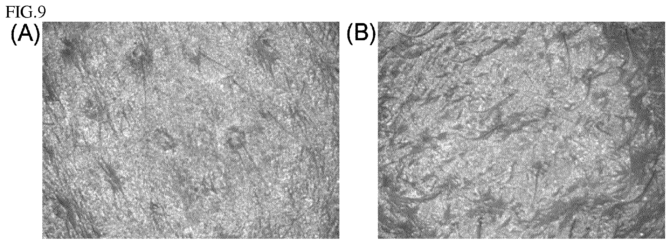

After a lapse of 5 hours of the application of the cosmetic material, the picture of the skin without the pasted base material film and the picture of the skin on which the base material film was pasted were taken and shown in Fig. 9 (A) and Fig. 9 (B), respectively.

According to Fig. 9, when a makeup cosmetic was applied on the skin to which the base material film was not pasted, the foundation powder fell into the pores, and it was difficult to conceal pores. On the other hand, it was reconfirmed that an excellent roughness correction effect, without noticeable pores, could be realized by applying a makeup cosmetic on the skin to which the base material film was pasted.

On the other hand, the present inventors prepared thin films containing base material films (support body: PET mesh, nylon mesh, or non-woven cloth), by the (micro)gravure method, having the thickness other than the above-described thickness (300 nm (poly-L-lactic acid film 200 nm + acetylated hyaluronic acid 100 nm) and 400 nm (poly-L-lactic acid film 200 nm + acetylated hyaluronic acid 200 nm)) and pasted the base material film on the skin; as a result, all of them were excellent in the roughness correction effect.

Accordingly, the sun care cosmetic for use in the method of the present invention is characterized by having a step of pasting the base material film surface of a thin film on the skin, a step of removing the support body of the pasted thin film, a step of applying a sun care cosmetic on the skin, wherein the thin film consists of a base material film of the thickness of 10 to 500 nm and a support body.

Using the sun care cosmetic for use in the method of the present invention, the roughness correction effect is excellent and the makeup cosmetic and sun care cosmetic can be easily removed.

As a result of further investigation by the present inventors, it was clarified that even when the order of the step of pasting the base material film on the skin and the step of removing the support body of the thin film is reversed, it has a similar effect.

Subsequently, the present inventors observed the skin wherein a makeup cosmetic is applied on the skin to which the thin film, which was obtained by the above preparation method, was pasted.

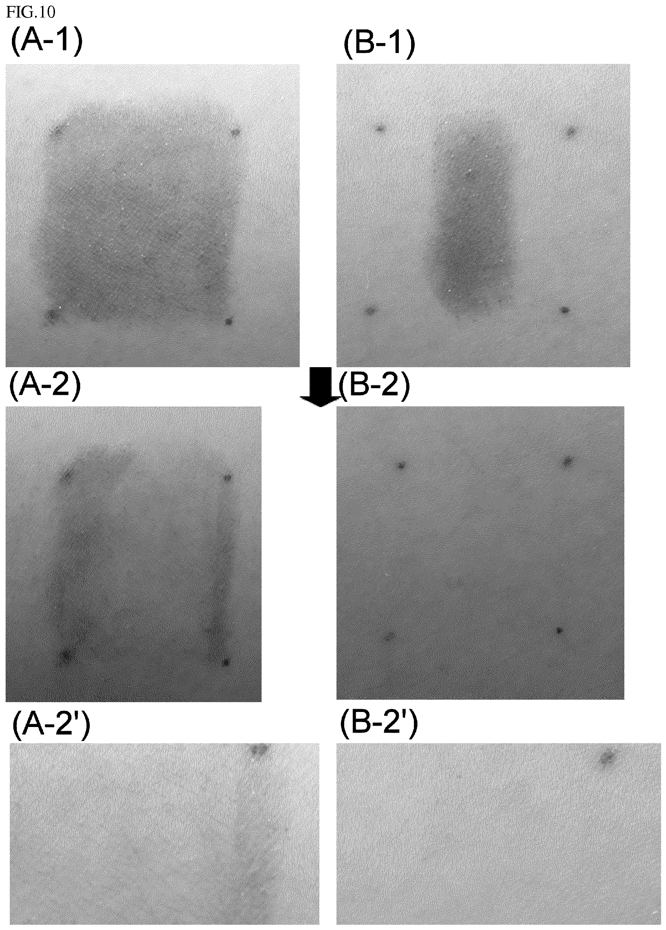

The pictures of the skins (A (control) and B), whose makeup was applied by the below method, are shown in Fig. 10 (A-1) and Fig. 10 (B-1), respectively. The pictures of the skins when the makeup was peeled off with cellophane tape are shown in Fig. 10 (A-2) and Fig. 10 (B-2), respectively. Fig. 10 (A-2') and Fig. 10 (B-2') are enlarged pictures of Fig. 10 (A-2) and Fig. 10 (B-2), respectively.

### • Skin A (control)

Eye shadow of the below-described Reference Example 2 was applied on the non-coated skin with an eye shadow tip.

### • Skin B

A little water was put on the skin, a base material film was pasted, and the support body was peeled off. After waiting for 1 minute so that the base material film becomes dry, the eye shadow of the below-described Reference Example 2 was applied with an eye shadow tip.

### Reference Example 2: Eye shadow

| | |
|---|---|
| Talc | 45 mass% |
| Mica | 15 |
| Sericite | 5 |
| Pigment | 15 |
| Pearlescent pigment | 10 |
| Liquid paraffin | 6 |
| Dimethylpolysiloxane | 2 |
| Antioxidant | 2 |
| Preservative | proper quantity |

According to Fig. 10, it was found that the makeup cosmetic on the base material film could easily be removed, from the skin on which the base material film was pasted, by peeling off the base material film. According to the enlarged pictures, the cosmetic material remained in the texture of skin A; however, it was thoroughly removed from skin B.

Subsequently, the present inventors investigated the percutaneous absorption effect of the cosmetic material by applying the skin care cosmetic (tranexamic acid solution) before pasting the thin film obtained by the above-described preparation method.

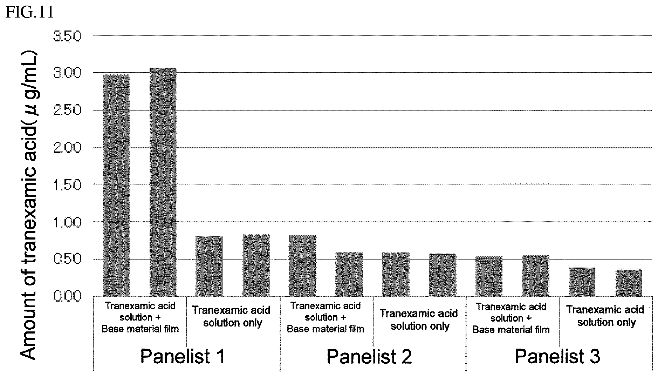

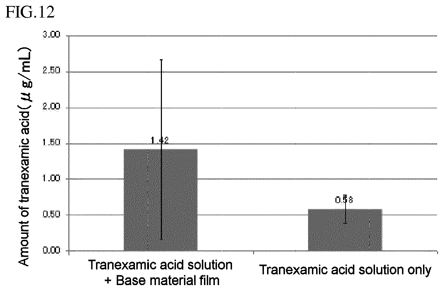

The stratum corneum of the skin on which only tranexamic acid solution, which is the control, was applied (only tranexamic acid solution) and the stratum corneum of the skin on which a thin film was pasted after the application of tranexamic acid solution (tranexamic acid solution + a thin film) were collected by the below method. Then, tranexamic acid was extracted from the collected stratum corneum into water, and it was assayed by liquid chromatography-mass spectrometric analysis (LC-MS). The results of the test, which was carried out two times by three panelists are shown in Fig. 11, and the average values of the results are shown in Fig. 12.

### • Collection method of the stratum corneum

It was carried out in a constant-temperature constant-humidity room at 22 °C and the relative humidity of 20%.

At first, the inner side of the left forearm was cleansed with soap for three panelists.

Subsequently, 2% tranexamic acid aqueous solution was applied on two test sites, respectively (quantity of application: 2µl/cm²), and they were dried. After being dried, water was applied.

One test site (control) was dried as it is. On the other test site, a base material film was pasted.

Then, they were placed at rest in a constant-temperature constant-humidity room for 6 hours. After 6 hours, each test site was cleansed with soap. On this occasion, the base material film was also washed away with water.

Then, the stratum corneum at each test site was collected with Aron Alpha.

According to Fig. 11 and Fig. 12, the percutaneous absorption effect of the active component (tranexamic acid) was found to increase by pasting the base material film after the application of a skin care cosmetic (tranexamic acid solution).

That is, tranexamic acid crystallizes in a dry environment when only tranexamic acid solution is present, and it is difficult to be percutaneously absorbed. However, it was suggested that the crystallization does not take place when a base material film is pasted and the penetration amount into the skin increases.

Subsequently, the present inventors investigated the roughness correction effect and moisturizing effect for the rough stratum corneum by applying a skin care cosmetic (moisturizing lotion) before pasting the thin film obtained by the above preparation method.

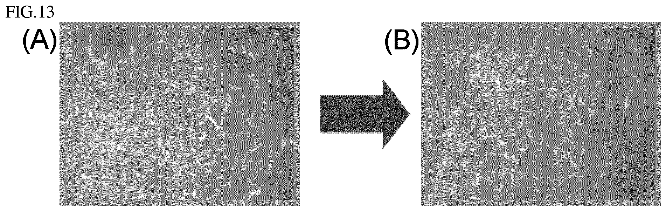

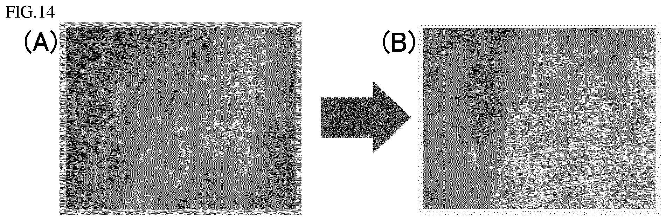

The pictures for the skin (B) wherein the below cosmetic method was applied to the skin (A) are shown in Fig. 13(B) (control) and Fig. 14(B), respectively.

### • Cosmetic method

It was carried out in a constant-temperature constant-humidity room at 23 °C and the relative humidity of 10%.

At first, the moisturizing lotion shown in the below-described Reference Example 3 was applied on the two test sites at the inner side of a panelist's left forearm (quantity of application: 2µl/cm²).

One test site was allowed to stand for 8 hours (control) as it is. The other test site was allowed to stand for 8 hours after a base material film was pasted.

### Reference Example 3: Moisturizing liquid

| | |
|---|---|
| Dimethylpolysiloxane | 5 mass% |
| Ethanol | 5 |
| Glycerin | 4 |
| Dipropyleneglycol | 5 |
| Polyethylene glycol 1500 | 2 |
| Pentaerythrityl tetra-2-ethylhexanoate | 2 |
| Self-emulsified glyceryl monoisostearate | 0.5 |
| Polyoxyethylene glyceryl isostearate | 0.5 |
| Polyoxyethylene glyceryl monostearate | 0.2 |
| Phenoxyethanol | proper quantity |
| Disodium edetate dihydrate | proper quantity |
| Xanthane gum | 0.1 |
| Acrylate/alkyl methacrylate copolymer | 0.1 |
| Carboxyvinylpolymer | 0.1 |
| Ion-exchanged water | balance |

When we compare Fig. 13 (A), Fig. 13 (B) and Fig. 14 (A), Fig. 14 (B), Fig. 14 wherein a base material film was pasted shows less sections where the stratum corneum is detached and white-looking because of skin roughness.

Thus, the roughness correction effect and moisturizing effect can also be maintained over a long time by the use of the cosmetic method of the present invention.

### DESCRIPTION OF THE NUMERALS

10: Thin film
12: Base material film
14: Support body
16: Stratum corneum
18: Epidermis
20: Makeup cosmetic and/or sun care cosmetic
22: Skin care cosmetic

## Claims

1. A sun care cosmetic for use in a method of protecting the skin from UV rays having a step of removing a support body of a thin film, subsequently a step of pasting a base material film on the skin, and subsequently a step of applying a sun care cosmetic on the base material film, wherein the thin film consists of a base material film of the thickness of 10 to 500 nm and a support body, wherein a film of hyaluronic acid, acetylated hyaluronic acid or sodium hyaluronate is supported on the base material film.

2. The sun care cosmetic for use in a method of protecting the skin from UV rays according to claim 1, wherein the base material film comprises one or more kinds of polylactic acid, polyglycolic acid, polycaprolactone, a copolymer thereof, and acrylic urethane copolymer.

3. The sun care cosmetic for use in a method of protecting the skin from UV rays according to claim 1 or 2, wherein the support body is a water-soluble polymer film or a cloth.

4. The sun care cosmetic for use in a method of protecting the skin from UV rays according to any of claims 1 to 3, wherein the skin has roughness.

## Patentansprüche

1. Sonnenschutzkosmetikum zur Verwendung bei einem Verfahren zum Schützen der Haut gegenüber UV-Strahlen mit einem Schritt des Entfernens eines Trägerkörpers aus einem dünnen Film, darauffolgend einem Schritt des Klebens eines Grundmaterialfilms auf die Haut, und nachfolgend einem Schritt des Aufbringens eines Sonnenschutzkosmetikums auf den Grundmaterialfilm, wobei der dünne Film aus einem Grundmaterialfilm der Dicke von 10 bis 500 nm und einem Trägerkörper besteht, wobei ein Film von Hyaluronsäure, acetylierter Hyaluronsäure oder Natriumhyaluronat auf dem Grundmaterialfilm getragen wird.

2. Sonnenschutzkosmetikum zur Verwendung bei einem Verfahren zum Schützen der Haut gegenüber UV-Strahlen nach Anspruch 1, wobei der Grundmaterialfilm eine oder mehrere Arten von Polymilchsäure, Polyglykolsäure, Polycaprolacton, einem Copolymer hiervon und Acryl-Urethan-Copolymer umfasst.

3. Sonnenschutzkosmetikum zur Verwendung bei einem Verfahren zum Schützen der Haut gegenüber UV-Strahlen nach Anspruch 1 oder 2, wobei der Trägerkörper ein wasserlöslicher Polymerfilm oder ein Stoff ist.

4. Sonnenschutzkosmetikum zur Verwendung bei einem Verfahren zum Schützen der Haut gegenüber UV-Strahlen nach irgendeinem der Ansprüche 1 bis 3, wobei die Haut Rauigkeit aufweist.

## Revendications

1. Un cosmétique écran solaire pour usage dans un procédé pour la protection de la peau des rayons UV comportant une étape de remuement d'un corps de support d'un film mince, ensuite une étape de collage d'un film de matériau de base sur la peau, et ensuite une étape d'application d'un cosmétique écran solaire sur le film de matériau de base, dans lequel le film mince consiste d'un film de matériau de base d'une épaisseur de 10 à 500 nm et d'un corps de support, dans lequel un film d'acide hyaluronique, d'acide hyaluronique acétylé et ou de l'hyaluronate de sodium est supporté sur le film de matériau de base.

2. Le cosmétique écran solaire pour usage dans un procédé pour la protection de la peau des rayons UV selon la revendication 1, dans lequel le film de matériau de base comprend une ou plusieurs sortes d'acide polylactique, d'acide polyglycolique, de polycaprolactone, d'un copolymère de ceux-ci, et d'un copolymère d'uréthane acrylique.

3. Le cosmétique écran solaire pour usage dans un procédé de protection de la peau des rayons UV selon la revendication 1 ou 2, dans lequel le corps de support est un film de polymère soluble dans de l'eau ou un tissu.

4. Le cosmétique écran solaire pour usage dans un procédé de protection de la peau des rayons UV selon l'une quelconque des revendications 1 à 3, dans lequel la peau à des rugosités.
